Europäisches Patentamt

European Patent Office

Office européen des brevets

(19)

(11) Veröffentlichungsnummer: **0 268 973**
**A2**

(12) # EUROPÄISCHE PATENTANMELDUNG

(21) Anmeldenummer: 87116917.3

(22) Anmeldetag: 17.11.87

(51) Int. Cl.⁴: **A61K 39/395**

(30) Priorität: 27.11.86 DE 3640513

(43) Veröffentlichungstag der Anmeldung:
01.06.88 Patentblatt 88/22

(84) Benannte Vertragsstaaten:
AT BE CH DE ES FR GB GR IT LI LU NL SE

(71) Anmelder: **Biotest Pharma GmbH**
**Flughafenstrasse 4**
**D-6000 Frankfurt 73(DE)**

(72) Erfinder: **Rudnick, Dieter, Dr. Dipl.-Chem.**
**Görlitzer Strasse 16**
**D-6078 Rödermark(DE)**
Erfinder: **Kothe, Norbert, Dr. Dipl.-Chem.**
**Friedrich-Ebert-Strasse 21**
**D-6242 Kronberg/Ts.(DE)**
Erfinder: **Dichtelmüller, Herbert, Dr.**
**Rossertstrasse 14**
**D-6231 Sulzbach/Ts.(DE)**
Erfinder: **Piechaczek, Detlef, Dr. Dipl.-Chem.**
**Darmstädter Strasse 54**
**D-6115 Münster(DE)**
Erfinder: **Stephan, Wolfgang, Dr. Dipl.-Chem.**
**Philipp-Holzmann-Strasse 84**
**D-6072 Dreieich(DE)**
Erfinder: **Schleussner, Hans, Dr. Dipl.-Chem.**
**Nansenring 26**
**D-6000 Frankfurt/M. 70(DE)**

(74) Vertreter: **Beil, Hans Chr., Dr. et al**
**Beil, Wolff und Beil, Rechtsanwälte**
**Adelonstrasse 58 Postfach 80 01 40**
**D-6230 Frankfurt am Main 80(DE)**

(54) **Verfahren zur Herstellung eines virussicheren, lagerstabilen und intravenös verträglichen Immunglobulin-G-Präparates.**

(57) Die Erfindung betrifft ein Verfahren zur Herstellung eines virussicheren, lagerstabilen und intravenös verträglichen Immunglobulin-G-Präparates, bei dem man aus einem von Gerinnungsfaktoren befreiten Plasma oder einer IgG-haltigen Plasma-oder Serumfraktion unter Anwendung einer Diafiltration oder einer Gelfiltration das Fällungsmittel entfernt und die gewünschte Ionenzusammensetzung einstellt, die erhaltene Proteinlösung zur Abtrennung des IgG von den restlichen Proteinen einer Fraktionierung an einem Ionenaustauscher unterwirft, in der erhaltenen IgG-Lösung die proteolytischen Enzyme durch Affinitätschromatographie an einem mit einem Farbstoff gekoppelten Sorbens entfernt und/oder durch Zusatz von Antithrombin III inhibiert, die so stabilisierte IgG-Lösung bei einer Proteinkonzentration von 20 bis 60 g/l und einem pH-Wert von 6,0 bis 8,0 mit 0,03 bis 0,07% β-Propiolacton behandelt und nach Verdünnen auf eine Proteinkonzentration von 5 bis 20 g/l einer Bestrahlung mit UV-Licht unterwirft, die sterilisierte, verdünnte IgG-Lösung zur Entfernung von niedermolekularen Substanzen nochmals diafiltriert oder gelfiltriert und die so stabilisierte und sterilisierte Lösung auf den gewünschten Proteingehalt einstellt und sterilfiltriert.

## Verfahren zur Herstellung eines virussicheren, lagerstabilen und intravenös verträglichen Immunglobulin-G-Präparates.

Die Erfindung betrifft das in den Patentansprüchen beschriebene Verfahren zur Herstellung eines virussicheren, lagerstabilen und intravenös verträglichen Immunglobulin-G-Präparates.

Für die Therapie von angeborenen oder erworbenen Immunmangelzuständen werden schon seit über 30 Jahren Immunglobulinpräparate angewandt, die aus humanem Plasma durch Fraktionierung gewonnen wurden. Diese über eine Fraktionierung mit kaltem Ethanol (Cohn-Fraktionierung) gewonnenen IgG-Präparate waren jedoch nur intramuskulär anwendbar, da bei intravenöser Applikation schwerste Nebenreaktionen beobachtet wurden. Da intravenös anwendbare Immunglobulinpräparate eine verbesserte Wirksamkeit aufweisen, wurden auch bereits Verfahren beschrieben, um aus alkoholfraktioniertem Immunglobulin i.v.-verträgliche Präparate herzustellen. Bei diesen Verfahren, die in einer Übersicht in Krankenhauspharmazie, 6. Jahrg., Nr. 5 (1985), S.226 bis 231 beschrieben sind, wurde das IgG-Molekül enzymatisch gespalten, z.B. mit Plasmin, oder chemisch modifiziert, z.B. mit ß-Propiolacton, oder es wurde versucht, die Bildung antikomplementärer Aggregate durch spezifische Fällungs-, Absorptions-und Chromatographieverfahren und Zusatz von Stabilisatoren zu verhindern bzw. die Aggregate zu entfernen und nicht modifiziertes IgG herzustellen.

Sowohl enzymatisch abgebaute wie chemisch modifizierte Immunglobuline (IgG) können nicht alle Funktionen des nativen Immunglobulins erfüllen, da die entsprechenden Verfahren eine Veränderung der Eigenschaften des Proteins, wie verminderte Halbwertszeit und Wirksamkeit oder verzögerten Wirkungseintritt bewirken.

Diese Nachteile haben zur Entwicklung alternativer Fraktionierverfahren für Immunglobuline geführt. Die Fällung mit Kolloiden wie Polyethylenglykol (PEG) oder Polyethylenglykol (PEG)/Hydroxyethylstärke (HES) führt dazu, daß nicht entferntes Kolloid infundiert wird; andererseits ist die PEG-Fällung als Verfahren zur Virusanreicherung bekannt. Auch läßt sich mit dieser Fraktioniermethode die hohe antikomplementäre Aktivität nicht beseitigen.

Zur Isolierung von Immunglobulin G aus Humanplasma wurde von Baumstark u.a. in Archives of Biochemistry and Biophysics 108 (1984), S. 514-522 ein Verfahren beschrieben, das Ionenaustauscher, insbesondere Anionenaustauscher wie DEAE-Sephadex, für diese Trennung in einem diskontinuierlichen Verfahren verwendet. Von Suomela u.a. wurde in "Separation of Plasma Proteins", herausgegeben von Curling, Pharmacia Fine Chemicals 1983, S. 127-130 ein Verfahren beschrieben, bei dem u.a. eine Gelfiltration, eine Chromatographie an einem Anionenaustauscher und eine Chromatographie an einem Kationenaustauscher für die Isolierung von Immunglobulin G aus Plasma eingesetzt wurden.

Von Friedli und Kistler wurde in "Methods of Plasma Protein Fractionation", herausgegeben von Curling, Academic Press 1980, S. 203-210 beschrieben, daß man durch Gelfiltration Ethanol und Salze aus einer Albuminlösung entfernen kann. In der gleichen Druckschrift wurde von Martinache und Henon auf S. 223-235 die Anwendung der Ultrafiltration zur Reinigung von IgG-Lösungen unter Entfernung von Ethanol und Salzen beschrieben.

Von Suomela wurde in der gleichen Druckschrift auf S.107 bis 116 darauf hingewiesen, daß im Handel erhältliche IgG-Präparate üblicherweise nicht lagerstabil sind, weil sie proteolytische Enzyme, z.B. Plasminogen, enthalten, die einen Abbau des IgG-Moleküls bewirken. Suomela versuchte, das Plasminogen aus einem mittels Ionenaustauscherchromatographie erhaltenen IgG-Präparat durch Chromatographie an Lysin-Agarose zu entfernen. Er stellte jedoch fest, daß dieses Verfahren nicht ausreichte, um alle proteolytischen Enzyme zu entfernen. Von Suzuki und Takahashi wurde in Methods in Enzymeology 34 (1974) auf S. 432 bis 435 die Gewinnung von reinem Präkallikrein aus einer Pseudoglobulinfraktion durch Adsorption an Arginin-Agarose beschrieben.

Die Behandlung einer Immunglobulin-Fraktion mit spezifischen Adsorbentien reicht nicht zur Entfernung der Gesamtproteasenaktivität aus, da diese Fraktionen in Abhängigkeit vom Ausgangsmaterial variierende Mengen der verschiedensten Enzyme wie Präkallikrein, Kallikrein, Plasminogen, Plasmin, Thrombin oder Faktor XI, enthalten können.

Darüberhinaus können allgemein durch Applikation von Blut, Humanplasma oder Plasmafraktionen pathogene Viren übertragen werden. Hier sind besonders Hepatitis B-, Hepatitis Non-A/Non-B-sowie AIDS-Viren hervorzuheben, die zu lebensbedrohlichen Erkrankungen führen können.

Es sollte deshalb für gereinigte Plasmaproteinfraktionen unabdingbar sein, ein Sterilisationsverfahren durchlaufen zu haben, das eine ausreichende Virusinaktivierung bewirkt. Dies gilt besonders dann, wenn die Plasmafraktionen aus Pools von mehr als 1000 Spendern hergestellt werden.

Für Plasma und einige Plasmaproteinfraktionen, wie für Gerinnungspräparate - z.B. Faktor VIII, Faktor IX und Fibrinogen - sind Sterilisationsverfahren bekannt. Hierbei wurden z.B. eine Hitzebehandlung in Lösung, ein Erhitzen in lyophilisiertem Zustand, ein Erhitzen unter Dampfdruck sowie eine Sterilisation mit Detergentien und speziell für die Sterilisation von Plasma als Ausgangsmaterial für die Herstellung von Gerinnungspräparaten in der DE-PS 30 33 932 eine kombinierte Behandlung mit $\beta$-Propiolacton und UV-Bestrahlung beschrieben.

Für die Herstellung von Immunglobulinpräparaten wurde eine Sterilisation bisher als nicht notwendig erachtet, da Immunglobuline, die durch Cohn-Fraktionierung hergestellt wurden, bisher als virussicher galten. In The Lancet 1986, S.322 und S.581-582 wurde jedoch die Übertragung von Hepatitis B, Hepatitis Non-A/Non-B, Hepatitis A sowie möglicherweise von AIDS durch IgG-Präparate beschrieben. Möglicherweise steht die Übertragung von Hepatitis Non-A/Non-B im Zusammenhang mit chromatographisch gereinigten Immunglobulinen. Deshalb erscheint es dringend notwendig, auch Immunglobuline einem Sterilisationsschritt zu unterziehen.

Die nach chromatographischen Verfahren hergestellten Immunglobulinpräparate sind in flüssigem Zustand nicht stabil und mit einem Risiko der Virusübertragung behaftet. Eine Hitzesterilisation dieser Präparate ist nicht möglich, da diese Behandlung zur Ausbildung von Aggregaten führt und die antikomplementäre Aktivität erhöht. Die Behandlung mit Detergentien wie Sorbimacrogololeat erfordert großen Aufwand, um die Detergentien aus dem Präparat zu entfernen. Die Umsetzung mit $\beta$-Propiolacton führt, wie von Stephan und Dichtelmüller in Arzneim.-Forsch./Drug Res. 33 (II), 9, 1230-1231 (1983) gezeigt wurde, unter den bekannten Bedingungen zwar zu einem virussicheren Präparat, jedoch auch zu einer chemischen Modifizierung des Proteins.

Der Erfindung liegt die Aufgabe zugrunde, ein in technischem Maßstab wirtschaftlich durchführbares Verfahren zur Herstellung eines unmodifizierten, intakten, hochreinen, virussicheren, lagerstabilen Immunglobulin-G-Präparates, das frei von antikomplementärer Aktivität und damit intravenös verträglich ist,durch Anreicherung und mehrstufige Reinigung eines von Gerinnungsfaktoren befreiten Plasmas oder einer IgG-haltigen Plasma-oder Serumfraktion unter Behandlung mit Ionenaustauschern und Ultrafiltration bereitzustellen.

Diese Aufgabe wird erfindungsgemäß dadurch gelöst, daß man

(a) aus dem von Gerinnungsfaktoren befreiten Plasma oder der IgG-haltigen Plasmafraktion unter Anwendung einer Diafiltration oder einer Gelfiltration das Fällungsmittel entfernt und die gewünschte Ionenzusammensetzung einstellt,

(b) die erhaltene Proteinlösung zur Abtrennung des IgG von den restlichen Proteinen einer Fraktionierung an einem Ionenaustauscher unterwirft,

(c) in der erhaltenen IgG-Lösung die proteolytischen Enzyme durch Affinitätschromatographie an einem mit einem Farbstoff gekoppelten Sorbens entfernt und/oder durch Zusatz von Antithrombin III inhibiert,

(d) die so stabilisierte IgG-Lösung bei einer Proteinkonzentration von 20 bis 60 g/l und einem pH-Wert von 6,0 bis 8,0 mit 0,03 bis 0,07% $\beta$-Propiolacton behandelt und nach Verdünnen auf eine Proteinkonzentration von 5 bis 20 g/l einer Bestrahlung mit UV-Licht unterwirft,

(e) die sterilisierte, verdünnte IgG-Lösung zur Entfernung von niedermolekularen Substanzen nochmals diafiltriert oder gelfiltriert und

(f) die so stabilisierte und sterilisierte Lösung auf den gewünschten Proteingehalt von 2 bis 16% einstellt und sterilfiltriert.

Das erfindungsgemäße Verfahren kann sowohl mit humanen als auch mit tierischen Ausgangsmaterialien durchgeführt werden.

Als Ausgangsmaterial kann ein von den Gerinnungsfaktoren befreites Plasma, Serum, eine durch Cohn-Fraktionierung isolierte Gammaglobulinfraktion (Cohn-Paste II oder II/III), die bei der großtechnischen Herstellung von Humanalbumin in großen Mengen anfällt, oder auch eine durch Salzfällung (z.B. mit Ammoniumsulfat) isolierte rohe Gammaglobulinfraktion eingesetzt werden. Diese Ausgangsmaterialien können sowohl von normalen Spenderpools als auch von ausgewählten Spendern mit hohen Antikörpertitern gegen virale und bakterielle Antigene gewonnen werden.

Vorzugsweise werden als Ausgangsmaterial des erfindungsgemäßen Verfahrens ein von den Gerinnungsfaktoren befreites Plasma oder Cohn-Paste II oder II/III eingesetzt.

Die Abtrennung der wertvollen Gerinnungsfaktoren aus Plasma, die selbst von großem therapeutischem Nutzen sind, andererseits jedoch die weitere Fraktionierung des Plasmas stören, ist bekannt. So wird beispielsweise das aus einem Auftauprozeß gewonnene Kryopräzipitat als Ausgangsmaterial für die Herstellung von Faktor VIII-Konzentraten verwendet, und die Faktoren II, VII, IX und X (PPSB-Komplex) werden durch Adsorption an einem Anionenaustauscher isoliert und in gereinigter Form ebenfalls therapeutisch eingesetzt.

Zur Gewinnung des bevorzugten Ausgangsmaterials für das erfindungsgemäße Verfahren wird daher ein nach den Richtlinien des Blutspendewesens mittels Plasmapherese gewonnenes Plasma bei +4°C aufgetaut und das Kryopräzipitat durch Zentrifugation entfernt. Die Gerinnungfaktoren des PPSB-Komplexes werden durch Adsorption an einem Anionenaustauscher, z.B. einem mit Diethylaminoethylgruppen substituierten vernetzten Dextran, abgetrennt. Das so behandelte Plasma kann vorzugsweise zur Entfernung des restlichen Fibrinogens einer Fällung mit Ethanol unterzogen werden. Dieser Schritt ist nicht unbedingt notwendig, vermeidet aber bei den nachfolgenden chromatographischen Schritten eine Verunreinigung der Säulenmaterialien. Die Bedingungen bei diesem Verfahrensschritt, nämlich eine Ethanolkonzentration von 8 bis 11 % und ein pH-Wert von 4,5-6,5, werden so gewählt, daß keine Bildung von Aggregaten in der Immunglobulinfraktion auftritt.

Ein so behandeltes Plasma zeigt folgende Zusammensetzung:

Proteingehalt 40-55 g/l, IgG-Gehalt 5,0-7,5 g/l,
Alkoholkonzentration 8 - 11 %,
$\alpha_1$-Globulin 2,5 - 3,5%, $\alpha_2$-Globuline 7,0-9,0%,
$\beta$-Globulin 7,0-9,0%, $\gamma$-Globulin 11-15 %,
Albumin 62 - 68% (Elektrophorese mit Celluloseacetatfolie).

Dieses von Gerinnungsfaktoren befreite Plasma oder auch die angereicherte Immunglobulinfraktion enthalten aufgrund der Vorbehandlung in beträchtlichen Mengen Alkohol (Ethanol) oder andere Fällungsmittel. Außerdem ist das Ionenmilieu durch die natürliche Zusammensetzung von Plasma oder durch die Vorbehandlung vorgegeben.

Zur weiteren Verarbeitung ist es notwendig, den Alkohol bzw. das Fällungsmittel zu entfernen und ein definiertes Ionenmilieu einzustellen. Im nächsten Verfahrensschritt erfolgt daher die Abtrennung des Alkohols bzw. des Fällungsmittels und die Einstellung der gewünschten Ionenzusammensetzung. Dies kann durch eine Diafiltration mit dem 5-10-fachen Austauschvolumen erfolgen. Hierzu werden Ultrafiltrationsmembranen mit einer Ausschlußgrenze von 10-100 000 Dalton verwendet, die sowohl als Flachmembran als auch als Hohlfaser angeordnet sein können. Die gewünschte Proteinkonzentration (20-30 g/l) wird am Ende des Prozesses eingestellt.

Vorzugsweise wird für diesen Verfahrensschritt eine Gelfiltration durchgeführt. Das Prinzip der Gelfiltration beruht darauf, daß man poröse Gelmaterialien verwendet, die niedermolekulare Substanzen, wie Alkohol und Salze retardieren, während hochmolekulare Stoffe, wie Plasmaproteine, die Gelmatrix ohne mit dieser in Wechselwirkung zu treten passieren. Dieses Prinzip wird verwendet, um niedermolekulare Substanzen abzutrennen und gleichzeitig, je nach Zusammensetzung der mobilen Phase, die gewünschte Ionenzusammensetzung einzustellen. Für die Gelfiltration eignen sich Gelmaterialien, die eine Ausschlußgrenze von 2-10 000 Dalton aufweisen, z.B. solche auf der Basis von vernetztem Dextran, von Mischpolymerisaten von N-Acryloyl-2-amino-2-hydroxymethyl-1,3-propandiol oder von Cellulose, wie Sephadex-G-25, Trisacryl GF 05 oder Cellufine GH 25.

Vor der Aufgabe der Proteinlösung wird das in einer Chromatographiesäule befindliche Gel mit dem gewünschten Puffersystem äquilibriert. Je nach weiterer Aufarbeitung an Anionen-oder Kationenaustauschern eignen sich Puffersysteme mit einer Molarität von 0,01-0,05 und einem pH-Wert von 4,5 - 8,5.

Die so behandelte Proteinlösung ist frei von Alkohol und störenden Salzen und setzt sich wie folgt zusammen:

Proteingehalt 20 - 30 g/l,
IgG-Gehalt 2,5 - 3,7 g/l,
$\alpha_1$-Globulin 2,5 - 3,5 %
$\alpha_2$-Globulin 7,0 - 9,0 %,
$\beta$-Globulin 7,0 - 9,0 %,
Albumin 62 - 68 %,
$\gamma$-Globulin 11 - 15 % (Elektrophorese mit Celluloseacetatfolie).

Diese Proteinlösung wird zur Abtrennung des IgG von den restlichen Proteinen einer Fraktionierung an Ionenaustauschern unterworfen. Beim Einsatz von Kationenaustauschern (z.B. mit Sulfopropylgruppen oder Carboxymethylgruppen substituierte Agarose oder Mischpolymerisate von N-Acryloyl-2-amino-2-hydroxymethyl-1,3-propandiol, wie S-Sepharose, SP-Trisacryl, CM-Sepharose) werden die Bedingungen so gewählt (Puffer 0,01-0,05molar,pH 4,5 - 6,5), daß die Immunglobulin-G-Fraktion an der Austauschermatrix gebunden wird, während alle anderen Plasmaproteine mit der mobilen Phase die Matrix ungehindert passieren können. Das gebundene Immunglobulin-G wird anschließend mit einem Elutionspuffer hoher Salzkonzentration 0,5 - 1,5 molar eluiert.

Die Ausbeute an Immunglobulin G beträgt nach diesem Verfahren etwa 60 %, die Immunglobulin-G-Fraktion enthält geringe Mengen an IgA und IgM.

Vorzugsweise wird deshalb die Fraktionierung des Plasmas zur Gewinnung der Immunglobulin-G-Fraktion an Anionenaustauschern (z.B. mit Diethylaminoethylgruppen substituierte Agarose, Mischpolymere von N-Acryloyl-2-amino-2-hydroxymethyl-1,3-propandiol, Cellulose oder Kieselsäure oder ein quartär mit Aminoethylgruppen substituiertes Polymerisat von Methacrylamid und N-Methylenbismethacrylamid enthaltenden Monomeren, wie DEAE-Sepharose, DEAE-Trisacryl, DEAE-Cellulose,DEAE-Spherosil, QAE-Accel oder QAM-Eupergit) durchgeführt. Das Puffermilieu wird in diesem Fall so gewählt (0,01 - 0,05 molar, pH 6,5-8,5), daß alle Plasmaproteine an die Anionenaustauschermatrix gebunden werden, die IgGFraktion jedoch, ohne mit dem Gelbett in Wechselwirkung zu treten, als reine Fraktion aufgefangen wird.

Nach dieser Methode läßt sich eine Immunglobulin-G-Fraktion in etwa 90%iger Ausbeute erhalten, die ohne Verunreinigungen von IgA, IgM und anderen Plasmaproteinen zu 100% aus $\gamma$-Globulin besteht (Elektrophorese mit Celluloseacetatfolie). Diese hochreine, verdunnte Immunglobulin-G-Fraktion wird zur Entfernung von Puffersubstanzen gegen physiologisch verträgliche Salzlösungen diafiltriert und zur Einstellung der gewünschten Proteinkonzentration mittels Ultrafiltration ankonzentrert. Hierzu eignen sich Ultrafiltrationsmembranen als Flach-oder Hohlfasermodul mit einer Ausschlußgrenze von 10 - 100 000 Dalton.

Die so oder auch auf andere bekannte Weise, z.B. durch Fällung, Gefrier-oder Sprühtrocknung und anschließende Solubilisierung oder durch Adsorption an Kationenaustauschern und nachfolgende Elution ankonzentrierte Immunglobulin-G-Lösung mit einem Proteingehalt von etwa 50 - 110 g/l ist jedoch noch nicht lagerstabil, da nach einiger Zeit Zersetzungsprodukte auftreten. Mit Hilfe des Enzymsubstrats S 22 88 (Kabi) konnte gezeigt werden, daß eine beträchtliche proteolytische Aktivität von bis zu 10 000 E/l in der Immunglobulin-G-Fraktion enthalten ist. Mit spezifischen Enzymsubstraten, d.h. Plasmin-, Plasminogen-, Thrombin-und Präkallikreinsubstraten, konnten Spuren der Enzyme nachgewiesen werden. Gängige Methoden zur Entfernung dieser Enzyme, wie Adsorption an Bariumsulfat, weißem Ton, Agar, kolloidaler Kieselsäure, Aktivkohle, Affinitätsaustauschern, Kationen-oder Anionenaustauschern sowie Zusätze von Aprotinin, Aminosäuren und Enzyminhibitoren wie Fluorverbindungen, führten zu keiner ausreichenden Abtrennung oder Inhibierung.

Erfindungsgemäß werden die proteolytischen Enzyme durch Affinitätschromatographie an einem mit einem Farbstoff gekoppelten Sorbens, z.B. Chromblau (Ciba), gekoppelt an Agarose, Cellulose oder ein Mischpolymerisat von N-Acryloyl-2-amino-2-hydroxymethyl-1,3-propandiol, wie Sepharose, Cellulose oder Trisacryl abgetrennt oder durch Zusatz von Antithrombin III inhibiert.

Die Effektivität der erfindungsgemäßen Affinitätschromatographie ist aus Tabelle I ersichtlich.Die dort untersuchten Ansätze 1 bis 5 wurden nach der Arbeitsweise des nachstehenden Beispiels 2 erhalten.

## Tabelle I

Proteolytische Aktivität von IgG-Präparaten nach
4-wöchiger Lagerung bei 37°C

| Ansatz | Proteolytische Aktivität E /1 | Spaltprodukte (HPLC) |
|---|---|---|
| 1 | 13 | < 1 % |
| 2 | 7 | < 1 % |
| 3 | 17 | < 1 % |
| 4 | 11 | < 1 % |
| 5 | 10 | < 1 % |

Die Effektivität eines erfindungsgemäßen Zusatzes von gereinigtem Antithrombin III zur Inhibierung der proteolytischen Enzyme ist aus Tabelle II ersichtlich. Die dort untersuchten Ansätze 1 bis 4 wurden nach der Arbeitsweise des nachstehenden Beispiels 1 erhalten.

## Tabelle II

Proteolytische Aktivität von IgG-Präparaten nach
4-wöchiger Lagerung bei 37°C

| Ansatz | Proteolytische Aktivität E /1 | Spaltprodukte (HPLC) |
|---|---|---|
| 1 | 7 | < 1 % |
| 2 | 11 | < 1 % |
| 3 | 4 | < 1 % |
| 4 | 10 | < 1 % |

Um eine lagerstabile IgG-Lösung zu erhalten, ist ein Zusatz von 0,5 bis 3 E/ml Antithrombin III erforderlich.

Die auf diese Weise gereinigte und stabilisierte Immunglobulin-G-Fraktion kann potentiell mit pathogenen Viren, wie Hepatitis B, Hepatitis Non-A/Non-B, Hepatitis A und HTLV-III/LAV kontaminiert sein.

Um ein virussicheres Immunglobulin-G-Präparat zu erhalten, wird daher erfindungsgemäß eine Sterilisation durchgeführt. In der EP-A 0 102 731 ist zwar beschrieben, daß mit Hilfe von Ionenaustauschern eine Reduktion von HBsAg möglich ist, eigene Versuche zeigen jedoch, daß diese Reduktion zur Erreichung eines virussicheren Produktes nicht ausreicht.

Setzt man einem erfindungsgemäß vorbereiteten Plasma vor der Fraktionierung an einem Ionenaustauscher $4,6 \times 10^{10}$ Kappa-Modellviren (Bakteriophagen) oder HBsAg, stellvertretend für Hepatitis-B-Viren, zu, verbleiben in der isolierten IgG-Fraktion $8,3 \times 10^3$ Viren pro ml. Entsprechende Versuchsergebnisse sind in Tabelle III zusammengestellt.

## Tabelle III

### Virusverteilung bei der chromatographischen
### Reinigung von IgG (DEAE-Trisacryl-Säule)

| Probe/ Behandlung | HBsAg (mg/ml) | Sendaivirus IE/ml | f-2 (Titer x Volume) | Kappa (Titer x Volume) |
|---|---|---|---|---|
| vor Chromatographie | 7,96 | $1,0 \times 10^2$ | $2,2 \times 10^{10}$ | $4,6 \times 10^{10}$ |
| nach Chromatographie | 0,017 | negativ | $8,3 \times 10^3$ | $8,3 \times 10^3$ |
| Titerreduktion | 99,79 % | $> 2,0 \log_{10}$ | $6,4 \log_{10}$ | $6,7 \log_{10}$ |
| Chromatographie-Eluat | 8,57 | $5,0 \times 10^2$ | $1,8 \times 10^8$ | $2,4 \times 10^9$ |

Diese Reduktion ist nicht ausreichend für ein virussicheres Immunglobulin-G-Präparat. Deshalb erhält das erfindungsgemäße Verfahren einen zusätzlichen Sterilisationsschritt. Zu diesem Zweck wird eine Kombination von $\beta$-Propiolacton und UV-Bestrahlung eingesetzt.

Die gereinigte Immunglobulin-G-Fraktion wird bei einer Proteinkonzentration von 20 - 60 g/l mit 0,03 - 0,07%, vorzugsweise mit 0,05%, $\beta$-Propiolacton ($\beta$PL) bei pH 6,0 - 8,0, vorzugsweise pH 7,2, behandelt. Nach einer Reaktionszeit von 3 - 24 h wird die Immunglobulinlösung auf einen Proteingehalt von 5 - 20 g/l verdünnt und einer UV-Bestrahlung in einer Rotationsdurchfluß-Apparatur unterzogen. Die so sterilisierte Lösung wird anschließend mittels bekannter Methoden auf 4 - 12 % Protein eingestellt. Wie aus Tabelle IV ersichtlich ist, werden durch das erfindungsgemäße Verfahren Modellviren mit ausreichender Sicherheit inaktiviert.

## Tabelle IV

Inaktivierung von Bakterien-Viren durch ßPL/UV
in chromatographisch gereinigtem IgG

| Probe | Phi-x 174 | Phi-e | Kappa |
|---|---|---|---|
| vor ßPL | $2,9 \times 10^7$ | $3,8 \times 10^7$ | $1,3 \times 10^9$ |
| Titerreduktion $(\log_{10})$ nach ßPL | 6,05 | 5,2 | 0,85 |
| Titerreduktion nach ßPL/UV kombiniert $(\log_{10})$ | > 7,5 | > 7,4 | 8,3 |

Die hierbei gefundene Virusinaktivierung von mindestens 7,5 $\log_{10}$ (Titerreduktion) entspricht einer ausreichenden Virussicherheit für Hepatitis B, Non-A/Non-B und AIDS.

Während die bekannte virusinaktivierende Wirkung von $\beta$-Propiolacton zu einer nachweisbaren chemischen Modifizierung der Immunglobuline führte, wird im erfindungsgemäßen Verfahren mit einer gegenüber dem bekannten Verfahren deutlich verminderten $\beta$-Propiolacton-Konzentration in Kombination mit einer UV-Bestrahlung überraschenderweise eine effektive Virusinaktivierung erzielt, ohne daß die Immunglobuline nachweisbar verändert oder in ihrer Aktivität beeinträchtigt werden. Dies wird in Tabelle V erläutert.

## Tabelle V

Einfluß von BPL/UV-Sterilisation auf chromatographisch gereinigtes IgG

| Probe | reziproke Antikörperaktivität (PHA) | | | | KBR (µl/mg) | Modifizierungsgrad (%) | Polymergehalt (%) |
|---|---|---|---|---|---|---|---|
| | E.coli | Ps.aerug. | Klebs. | Staph. | | | |
| vor Steri-lisation | 40 (12) | 80 (12) | 160 (19) | 20 (14) | > 135 | – | nicht nachweisbar |
| nach Steri-lisation | 40 (12) | 80 (15) | 160 (19) | 10 (8) | 15 | nicht nach-weisbar | < 1,0 |

Das erfindungsgemäß hergestellte Immunglobulin-G-Präparat zeichnet sich überraschenderweise durch die Kombination folgender Eigenschaften aus:

-Die Reinheit beträgt etwa 100 % an γ-Globulin (Elektrophorese mit Celluloseacetatfolie)
-Das Präparat ist frei von IgA und IgM
-Die Ausbeute beträgt bis zu 90 % an IgG
-Alle IgG-Subklassen sind im Präparat enthalten.

-Das Präparat enthält keine hochmolekularen Aggregate,weist deshalb eine niedrige unspezifische antikomplementäre Aktivität auf und ist aus diesem Grunde i.v-verträglich.

9

-Die Immunglobulinlösung ist virussicher sterilisiert, nativ und nicht modifiziert.

-Das Präparat enthält wirksame Proteasen in so geringer Menge, daß keine Spaltprodukte nachweisbar sind, und ist lagerstabil.

Diese Eigenschaften erfüllen alle Anforderungen, die an ein Immunglobulinpräparat zu stellen sind.

Die Erfindung wird durch nachstehende Beispiele erläutert.


Beispiel 1

A. Ein Pool aus 50 l frisch gefrorenem Humanplasma wurde bei +4°C aufgetaut, und der dabei verbleibende Niederschlag (Kryopräzipitat) wurde durch Zentrifugation abgetrennt. Der klare Überstand wurde zur Abtrennung von den Faktoren des PPSB-Komplexes mit vorgequollenem und äquilibriertem Ionenaustauscher (DEAE-Sephadex) versetzt und 1 h bei Raumtemperatur gerührt. Das Gel wurde über ein Filter entfernt und der Überstand wurde mit 9 Volumen-% Ethanol bei pH 5,3 versetzt. Nach einer Standzeit von 3 h bei -3°C wurde der Niederschlag, der zum Großteil aus Fibrinogen bestand, durch Zentifugation entfernt und eine Sterilfiltration durchgefuhrt.

B. Das vorstehend erhaltene, von Gerinnungsfaktoren befreite Plasma wurde zur Entfernung des Alkohols und zur Einstellung des Ionenmilieus auf 0,022 M Trishydroxymethyl-aminomethan (Tris)/HCl, pH 7,5, unter Verwendung einer mit 0,022 M Tris/HCl, pH 7,5 äquilibrierten Säule, die mit 22 l Sephadex-G-25 gefüllt war, gelfiltriert. Zur vollständigen Umpufferung des Plasmas waren 12 Gelfiltrationszyklen erforderlich. Die gesammelten Fraktionen mit einem Proteingehalt von 22 g/l wurden sterilfiltriert.

C. Aus dem so erhaltenen, umgepufferten Plasma wurde das IgG durch Anionenaustauscher-Chromatographie abgetrennt. Dazu wurde eine 10 l-Säule verwendet, die mit DEAE-Trisacryl-LS gefüllt und mit 0,022 M Tris/HCl, pH 7,5 äquilibriert worden war. In Portionen von 12 l wurde das umgepufferte Plasma an dem Anionenaustauscher fraktioniert, wobei alle Plasmaproteine außer IgG an den Austauscher gebunden wurden. Zur Aufarbeitung der gesamten Plasmamenge waren 6 Zyklen erforderlich.
Die gebundenen Plasmaproteine wurden jeweils mit 0,022 M Tris/HCl, 0,6 M NaCl pH 7,5 eluiert und für eine getrennte Verwendung gesammelt.

D. Die vereinigten IgG-Fraktionen mit einem Proteingehalt von 1,5 g/l und einem Volumen von 120 l wurden mit Hilfe einer Ultrafiltrationsanlage (Ausschlußgrenze 10 000 Dalton) auf einen Proteingehalt von 40 g/l ankonzentriert. Bei einem pH-Wert von 7,2 wurde die Lösung mit festem Kochsalz auf physiologisches Milieu eingestellt und mit 1,5 E/ml Antithrombin III versetzt, um etwa vorhandene proteolytische Enzyme zu inhibieren.

E. Die in Stufe D erhaltene IgG-Lösung wurde zwecks Sterilisation bei pH 7,2 mit $\beta$-Propiolacton (0,5 ml pro Liter Lösung) versetzt, wobei während der Reaktionszeit von 12 h der pH-Wert konstant gehalten wurde.

Danach verdünnte man mit physiologischer Kochsalzlösung auf einen Proteingehalt von 10 g/l und bestrahlte diese Lösung in einer UV-Rotationsdurchflußapparatur mit einer Durchflußgeschwindigkeit von 20 l/h und einer Intensität von 1 mWatt/cm² × Min.

F. Die so erhaltene sterilisierte IgG-Lösung wurde in 4 Zyklen zur Entfernung des Tris/HCl-Puffers unter Verwendung einer Säule, die mit 22 l Sephadex G-25 gefüllt und mit halbphysiologischer Kochsalzlösung äquilibriert worden war, gelfiltriert.

Nach Ankonzentrierung mit Hilfe einer Ultrafiltrationsanlage (Ausschlußgrenze 10 000 Dalton) auf einen Proteinwert von 50 g/l fügte man 0,150 Mol Glycin pro Liter zu und führte eine Sterilfiltration durch.

Diese so gewonnene Immunglobulin-G-Lösung hatte folgende Eigenschaften:

Ausbeute:       72 %
Protein:        58,8 g/l
IgG:            53,5 g/l
$\gamma$-Globuline:   100% (Elektrophorese mit Celluloseacetatfolie)
Komplementbindungsreaktion:   11 µl/mg Protein
Proteolytische
Aktivität:      7 E/l
Gelfiltration
(HPLC):         95 % Monomer
                5 % Dimer
                -% Polymer

-% Spaltprodukte

Modifizierung:   nicht nachweisbar

## Beispiel 2

Die Arbeitsweise von Beispiel 1, Stufen A bis C wurde wiederholt.

D. Die IgG-Fraktion mit einem Proteingehalt von 1,5 g/l wurde mit festem Kochsalz auf physiologische Salzkonzentration eingestellt.

Eine Chromatographie-Säule, gefüllt mit 3 1 Ciba-Chromblau-Trisacryl-LS wurde mit 0,022 M Tris/HCl, 0,15 M Kochsalz, pH 7,5 äquilibriert.

20 l der gereinigten, isotonen, verdunnten IgG-Lösung wurden mit einer Fließgeschwindigkeit von 6 l/h über das Gelbett gepumpt. Die von proteolytischen Enzymen befreite IgG-Lösung wurde mit einer Ultrafiltrationsanlage auf 40 g/l ankonzentriert.

Die Arbeitsweise von Beispiel 1, Stufen E und F wurde wiederholt.

Eigenschaften:

Ausbeute:        62 %

Protein:        53,5 g/l

IgG:        51,5 g/l

$\gamma$-Globuline:    99,1 % (Elektrophorese mit Celluloseacetatfolie)

Komplementbindungsreaktion:    15 $\mu$g/mg Protein

Modifizierung:   nicht nachweisbar

Gelfiltration

(HPLC):        93,4 % Monomer

5,89 % Dimer

0,66 % Polymer

-% Spaltprodukte

Proteolytische

Aktivität:    18 E/l

## Beispiel 3

Die Arbeitsweise von Beispiel 1, Stufe A wurde mit einem weiteren Pool von 50 l Humanplasma wiederholt, wobei jedoch die Behandlung mit Ethanol weggelassen wurde. Das von den Gerinnungsfaktoren befreite Plasma wurde nach Abfiltrieren des Ionenaustauschergels direkt der Stufe B (Gelfiltration) zugeführt, um das Ionenmilieu auf 0,022 M Tris/HCl pH 7,5 einzustellen. Sodann wurde die Arbeitsweise der Stufen B bis F von Beispiel 1 oder 2 wiederholt.

Eigenschaften:

Ausbeute:        70 %

Protein:        ·52,4 g/l

IgG:        51,8 g/l

$\gamma$-Globuline:    ~100 % (Elektrophorese mit Celluloseacetatfolie)

Komplementbindungsreaktion:    12 $\mu$l/mg Protein

Modifizierung:    nicht nachweisbar

Gelfiltration:

(HPLC)        85,9 % Monomer

14,1 % Dimer

-% Polymer

-% Spaltprodukte

Beispiel 4

A. Ein Kilogramm einer nach der Methode von Cohn durch Alkohol-Fraktionierung aus Humanplasma gewonnenen Rohgammaglobulinpaste (Paste II/III) wurde in 11 Litern 0,15 M Natriumdihydrogenphosphatpuffer, pH 5,2 gelöst. Der Proteingehalt dieser Lösung betrug 20 g/l. Zur Entfernung von unlöslichen Bestandteilen wurde zentrifugiert und anschließend filtriert.

B. Die erhaltene Lösung wurde nach der Arbeitsweise von Beispiel 1, Stufe B, gelfiltriert, um den Alkohol zu entfernen und das Ionenmilieu auf 0,022 M Tris/HCl pH 7,5 einzustellen.

Die in Stufe B erhaltene Lösung mit einem Proteingehalt von 15 g/l wurde nach der Arbeitsweise der Stufen C bis F der Beispiele 1 oder 2 weiterverarbeitet.

Eigenschaften:

Ausbeute: 56 %
Protein: 51,2 g/l
IgG: 49,7 g/l
γ-Globuline: 98 % (Elektrophorese mit Celluloseacetatfolie)
Komplementbindungsreaktion: 20 μl/mg Protein
Modifizierung: nicht nachweisbar
Gelfiltration
(HPLC): 93,1 % Monomer
6,9 % Dimer
-% Polymer
-% Spaltprodukte


Beispiel 5

Die Arbeitsweise von Beispiel 1, Stufen A bis F wurde wiederholt, wobei jedoch in Stufe C anstelle von DEAE-Trisacryl-LS Sepharose-Q eingesetzt wurde.

Ausbeute: 58,5 %
Protein: 48,7 g/l
IgG: 46,2 g/l
γ-Globuline: 98,9 % (Elektrophorese mit Celluloseacetatfolie)
Komplementbindungsreaktion: 19 μl/mg Protein
Modifizierung: nicht nachweisbar
Gelfiltration (HPLC): 93,5 % Monomer
6,5 % Dimer
-% Polymer
-% Spaltprodukte
Proteolytische
Aktivität: 17 E/l


Beispiel 6

Die Arbeitsweise von Beispiel 1, Stufen A bis F wurde wiederholt, wobei jedoch in Stufe C anstelle von DEAE-Trisacryl-LS DEAE-Spherosil eingesetzt wurde.

Ausbeute: 70 %
Protein: 49,5 g/l
IgG: 53,5 g/l
γ-Globuline: 100% (Elektrophorese mit Cellulose acetatfolie)
Komplementbindungsreaktion: 15 μl/mg Protein
Modifizierung: nicht nachweisbar
Gelfiltration
(HPLC): 94,5 % Monomer
5,5 % Dimer
-% Polymer

-% Spaltprodukte
Proteolytische
Aktivität:     9 E/l

## Beispiel 7

Die Arbeitsweise von Beispiel 1, Stufen A bis F wurde wiederholt, wobei jedoch in Stufe B anstelle von Sephadex-G-25 zur Alkoholentfernung und Umpufferung Trisacryl-GF 0,5 eingesetzt wurde.

Ausbeute:     70 %
Protein:      51,5 g/l
IgG:          50,2 g/l
γ-Globuline:   ~100 % (Elektrophorese mit Celluloseacetatfolie)
Komplementbindungsreaktion:      13 μl/mg Protein
Modifizierung:    nicht nachweisbar
Gelfiltration
(HPLC):        95 % Monomer
               5 % Dimer
               -% Polymer
               -% Spaltprodukte
Proteolytische Aktivität:     18 E/l

## Beispiel 8

A. Die Arbeitsweise von Beispiel 1, Stufe A wurde wiederholt.

B. Die in Stufe A erhaltene Lösung wurde zur Alkoholentfernung und Einstellung des Ionenmilieus an Sephadex-G-25, äquilibriert mit 0,025 M Natriumacetat, pH 5,8, gelfiltriert. Die gesammelten Fraktionen mit einem Proteingehalt von 22 g/l wurden sterilfiltriert.

C. Eine Chromatographie-Säule, die mit 12 SP-Trisacryl-LS gefüllt worden war, wurde mit einem 0,025 M Natriumacetatpuffer, pH 5,8, äquilibriert, und das in Stufe B erhaltene, umgepufferte Plasma wurde in 5 Zyklen von jeweils 19 l chromatographiert. Die an den Kationenaustauscher gebundene IgG-Fraktion wurde jeweils mit 0,025 M Natrium-Acetat, 0,6 M Kochsalz, pH 7,5 eluiert.

D. Die gesammelten IgG-Fraktionen wurden mit Hilfe einer Ultrafiltrationsanlage (Ausschlußgrenze 10 000 Dalton) auf einen Proteingehalt von 40 g/l ankonzentriert und gegen isotone Kochsalzlösung diafiltriert.

Die so erhaltene Lösung wurde entweder wie in Beispiel 1 mit Antithrombin III versetzt oder wie in Beispiel 2 einer Affinitätschromatographie an Ciba-Chromblau-Trisacryl-LS unterworfen.

Die weiteren Aufarbeitung erfolgte nach der Arbeitsweise der Stufen E und F von Beispiel 1.

Eigenschaften:

Ausbeute:     55 %
Protein:      52,3 g/l
IgG:          50,3 g/l
IgA:          0,8 g/l
IgM:          0,6 g/l
γ-Globuline:   97 % (Elektrophorese mit Celluloseacetatfolie)
Komplementbindungsreaktion:      12 μl/mg Protein
Modifizierung:    nicht nachweisbar
Gelfiltration
(HPLC):        91,5 % Monomer
               7 % Dimer
               1,5 % Polymer
               -% Spaltprodukte

## Ansprüche

1. Verfahren zur Herstellung eines virussicheren, lagerstabilen und intravenös verträglichen Immunglobulin-G-Präparates durch Anreicherung und mehrstufige Reinigung eines von Gerinnungsfaktoren befreiten Plasmas oder einer IgG-haltigen Plasma-oder Serumfraktion unter Behandlung mit Ionenaustauschern und Ultrafiltration, dadurch gekennzeichnet, daß man

(a) aus dem von Gerinnungsfaktoren befreiten Plasma oder der IgG-haltigen Plasmafraktion unter Anwendung einer Diafiltration oder einer Gelfiltration das Fällungsmittel entfernt und die gewünschte Ionenzusammensetzung einstellt,

(b) die erhaltene Proteinlösung zur Abtrennung des IgG von den restlichen Proteinen einer Fraktionierung an einem Ionenaustauscher unterwirft,

(c) in der erhaltenen IgG-Lösung die proteolytischen Enzyme durch Affinitätschromatographie an einem mit einem Farbstoff gekoppelten Sorbens entfernt und/oder durch Zusatz von Antithrombin III inhibiert,

(d) die so stabilisierte IgG-Lösung bei einer Proteinkonzentration von 20 bis 60 g/l und einem pH-Wert von 6,0 bis 8,0 mit 0,03 bis 0,07% β-Propiolacton behandelt und nach Verdünnen auf eine Proteinkonzentration von 5 bis 20 g/l einer Bestrahlung mit UV-Licht unterwirft,

(e) die sterilisierte, verdünnte IgG-Lösung zur Entfernung von niedermolekularen Substanzen nochmals diafiltriert oder gelfiltriert und

(f) die so stabilisierte und sterilisierte Lösung auf den gewünschten Proteingehalt von 2 bis 16% einstellt und sterilfiltriert.

2. Verfahren nach Anspruch 1, dadurch gekennzeichnet, daß man Stufe (a) als Diafiltration mit dem 5- bis 10-fachen Austauschvolumen an einer Membran mit einer Ausschlußgrenze von 10 bis 100 000 Dalton oder als Gelfiltration an einem Gelfiltrationsmaterial mit einer Ausschlußgrenze von 2 bis 10 000 Dalton durchführt, wobei ein Puffersystem mit einer Molarität von 0,01 bis 0,05 und einem pH-Wert von 4,5 bis 8,5 als Austauschmedium bzw. zur Äquilibrierung verwendet wird.

3. Verfahren nach Anspruch 2, dadurch gekennzeichnet, daß man als Gelfiltrationsmaterial ein vernetztes Dextrangel, ein Gel auf Basis von Mischpolymeren von N-Acryloyl-2-amino-2-hydroxymethyl-1,3-propandiol oder ein Cellulosegel verwendet.

4. Verfahren nach Anspruch 3, dadurch gekennzeichnet, daß man als Gelfiltrationsmaterial Sephadex-G-25, Trisacryl-GF-05 oder Cellufine-GH-25 verwendet.

5. Verfahren nach einem der Ansprüche 1 bis 4, dadurch gekennzeichnet, daß man in Stufe (a) das Gel mit einem Puffersystem mit einem pH-Wert von 6,5 bis 8,5 äquilibriert und in Stufe (b) die Fraktionierung mit einem Anionenaustauscher durchführt, der ebenfalls mit einem Puffersystem mit einer Molarität von 0,01 bis 0,05 und einem pH-Wert von 6,5 bis 8,5 äquilibriert wird.

6. Verfahren nach Anspruch 5, dadurch gekennzeichnet, daß man als Anionenaustauscher DEAE-Sepharose, DEAE-Trisacryl, DEAE-Cellulose, QAM-Eupergit oder QAE-Accel verwendet.

7. Verfahren nach einem der Ansprüche 1 bis 6, dadurch gekennzeichnet, daß man in Stufe (c) 0,1 bis 5 Einheiten/ml Antithrombin III zusetzt.

8. Verfahren nach einem der Ansprüche 1 bis 6, dadurch gekennzeichnet, daß man in Stufe (c) eine Affinitätschromatographie an einem mit Chromblau gekoppelten Sorbens durchführt.

9. Verfahren nach Anspruch 8, dadurch gekennzeichnet, daß man die Affinitätschromatographie an mit Chromblau gekoppeltem Trisacryl durchführt.

10. Verfahren nach einem der Ansprüche 1 bis 9, dadurch gekennzeichnet, daß man in Stufe (d) die Bestrahlung mit UV-Licht bei einer Intensität von 1 mWatt/cm$^2$ × Min. durchführt.

11. Verfahren nach einem der Ansprüche 1 bis 10, dadurch gekennzeichnet, daß man als Ausgangsmaterial ein Plasma verwendet, aus dem nach Entfernung des Kryopräzipitates die Gerinnungsfaktoren des PPSB-Komplexes durch Adsorption an einem Anionenaustauscher abgetrennt wurden.

12. Verfahren nach Anspruch 11, dadurch gekennzeichnet, daß man als Ausgangsmaterial ein von Gerinnungsfaktoren befreites Plasma verwendet, welches durch Fällung mit Ethanol von restlichem Fibrinogen befreit wurde.